Europäisches Patentamt

European Patent Office

Office européen des brevets

(11) Veröffentlichungsnummer: **0 449 799 A1**

(12) # EUROPÄISCHE PATENTANMELDUNG

(21) Anmeldenummer: **91890041.6**

(22) Anmeldetag: **28.02.91**

(51) Int. Cl.$^5$: **A61F 2/76, A61B 5/11**

(30) Priorität: **28.02.90 AT 476/90**

(43) Veröffentlichungstag der Anmeldung:
**02.10.91 Patentblatt 91/40**

(84) Benannte Vertragsstaaten:
**BE CH DE DK ES FR GB GR IT LI LU NL SE**

(71) Anmelder: **Otto Bock Orthopädische Industrie Besitz- und Verwaltungs-Kommanditgesellschaft Industriestrasse W-3428 Duderstadt (DE)**

(72) Erfinder: **Horvath, Eduard, Ing. Kaiserstrasse 39 A-1070 Wien (AT)**

(74) Vertreter: **Casati, Wilhelm, Dipl.-Ing. et al Patentanwälte Casati, Wilhelm, Dipl.-Ing. Itze, Peter, Dipl.-Ing. Amerlingstrasse 8 A-1061 Wien (AT)**

(54) **Verfahren und Vorrichtung zur Bestimmung von Gebrauchseigenschaften eines Prothesenschaftes.**

(57) Die Erfindung betrifft ein Verfahren zur Bestimmung von Gebrauchseigenschaften eines Prothesenschaftes (1) insbes. eines Beinprothesenschaftes (1), bei der Anprobe des Schaftes (1) unter Verwendung einer Tragvorrichtung (2) für den Schaft (1), wobei die um mindestens eine, bevorzugt jedoch um mindestens zwei einander schneidende, insbes. aufeinander senkrechte Achsen (5,6), schwenkbare Tragvorrichtung (2) des Prothesenschaftes (1), nachdem der Gliedmaßenstumpf, insbes. Beinstumpf, in den Prothesenschaft (1) eingesetzt wurde, mit der Achse bzw. dem Achsenschnittpunkt auf die Mitte (den Mittenbereich) des natürlichen Gelenkes des Gliedmaßen-, insbes. des Beinstumpfes, bzw. auf den Schwerpunkt des den Schaft (1) Probierenden, justiert wird, wonach der Prothesenschaft (1) mittels des Gliedmaßenstumpfes bewegt wird und die Grenzen der Bewegbarkeit des in den Gliedmaßen-, insbes. Beinprothesenschaft (1) eingesetzten Gliedmaßen-, insbes. Beinstumpfes koordinatenmäßig als Istwerte festgehalten werden und wobei die Istwerte mit genormten Sollwerten zwecks Beurteilung der Bewegungseinschränkung hinsichtlich ihrer Zulässigkeit verglichen werden und wobei aufgrund der solcherart jeder Stellung des Prothesenschaftes (1) zuordenbaren Abweichung zwischen Soll- und Istwert Änderungen beispielsweise im Stumpfbett vorgenommen werden, um die Abweichung zwischen Soll- und Istwert der Bewegbarkeit des Gliedmaßen-, insbesondere Beinprothesenschaftes (1) in dieser Stellung zu verringern.

EP 0 449 799 A1

FIG.1

## VERFAHREN UND VORRICHTUNG ZUR BESTIMMUNG VON GEBRAUCHSEIGENSCHAFTEN EINES PROTHESENSCHAFTES

Die Erfindung betrifft ein Verfahren zur Bestimmung von Gebauchseigenschaften eines Prothesenschaftes, insbes. eines Beinprothesenschaftes, bei der Anprobe des Schaftes unter Verwendung einer Tragvorrichtung für den Schaft.

In der Prothesentechnik sind eine Reihe von Aufbautechniken bekannt, die den Zweck verfolgen, eine Prothese, insbes. Beinprothese nach bestimmten Regeln zu konstruieren. Dabei stehen fast immer die technischen und kosmetischen Konstruktionsrichtlinien im Vordergrund. Unbestritten ist die Ansicht, daß für den Wert einer Prothese die Gebrauchseigenschaften des Prothesenschaftes von allergrößter Wichtigkeit sind, weil erst sie die Akzeptanz der Prothese ergeben. Die Erzielung dieser Gebrauchseigenschaften ist bislang der kunstvollen Intuition der Prothesentechniker überlassen. Diese bedienen sich inzwischen zwar bereits der Computertechnologie zur leichteren Darstellung, Veränderung und Wiederholung von Schaftformen, insbes. der Innenform des Schaftes, jedoch wird die Beurteilung der Gebrauchseigenschaften mit den bisher üblichen Verfahren der Interpretation verbaler Auskünfte des Patienten und visueller Beobachtungen des Prothesentechnikers überlassen. Hier setzt nun die Erfindung ein, mit dem Ziel, die Gebrauchseigenschaften eines Prothesenschaftes um das natürliche, dem Prothesenschaft zugeordnete Gelenk (bei Oberschenkelprothesen ist dies das Hüftgelenk) unter der Körperlast und auch unter simulierten äußeren Beanspruchungen zu ermitteln, um im Bedarfsfall gezielte Änderungen am Schaft vorzunehmen, um für den Prothesenträger individuell einen schmerzfreien, erträglichen und damit komfortablen Bewegungsbereich zu erzielen, der dem Bewegungsbereich der nichtamputierten Gliedmaße, bevorzugt des Beines, möglichst nahekommt.

Erreicht wird dies durch ein Verfahren, bei dem die um mindestens eine, bevorzugt jedoch um mindestens zwei einander schneidende, insbes. aufeinander senkrechte Achsen, schwenkbare Tragvorrichtung des Prothesenschaftes, nachdem der Gliedmaßenstumpf, insbes. Beinstumpf in den Prothesenschaft eingesetzt wurde, mit der Achse bzw. dem Achsenschnittpunkt auf die Mitte (den Mittenbereich) des natürlichen Gelenkes des Gliedmaßen-, insbes. des Beinstumpfes bzw. bei Beinprothesen auf den Schwerpunkt des den Schaft Probierenden justiert wird, wonach der Prothesenschaft mittels des Gliedmaßenstumpfes bewegt wird und die Grenzen der (schmerzfreien) Bewegbarkeit des in den Gliedmaßen-, insbes. Beinstumpfes eingesetzten Gliedmaßen-, insbes. Beinstumpfes koordinatenmäßig als Istwerte festgehalten werden und bei dem dann die Istwerte mit genormten Sollwerten zwecks Beurteilung der Bewegungseinschränkung hinsichtlich ihrer Zulässigkeit verglichen werden und aufgrund der solcherart jeder Stellung des Prothesenschaftes zuordenbaren Abweichung zwischen Soll- und Istwert, Änderungen insbes. im Stumpfbett vorgenommen werden, um die Abweichung zwischen Soll- und Istwert der Bewegbarkeit des Gliedmaßen-, insbes. des Beinprothesenschaftes in dieser Stellung zu verringern. Bei Anwendung des erfindungsgemäßen Verfahrens werden die Bewegungsbereiche vorzugsweise unter Zugrundelegung der gemessenen Grenzwinkel, innerhalb welchen die Bewegungen schmerzfrei erfolgen, dargestellt, wobei beispielsweise die Strahlen der Grenzlagen, die gedacht auf einer Bezugsebene, bei Beinprothesen auf die Gehebene, projiziert werden, eine für jeden Patienten und für jeden Schaft charakteristische typische Nutzfläche umgrenzen, die eine schnelle Beurteilung der Gebrauchseigenschaften, durch Vergleich mit "normalen" oder anderen als Normen festgelegten Flächen oder Markierungen erlaubt. Aus der relativen lage der ermittelten (Ist-))Nutzfläche zum Körper bzw. zur genormten (Soll-) Nutzfläche kann auf einfache Weise eine zweckmäßige, somit verbesserte Lage des Schaftes am Gliedmaßenstumpf errechnet und hernach vom Patienten die geänderte Position des Schaftes sogleich erprobt werden, die verbesserte Gebrauchseigenschaften des Prothese erwarten läßt. Damit ergibt sich die Möglichkeit, die Paßform des Stumpfbettes und die Position des Schaftes am Stumpf auf den tatsächlichen dynamischen Bewegungsbereich des Schaftes optimal abzustimmen. Um die Prothese fertigzustellen, werden dann die mechanischen Bestandteile der Prothese entsprechend den nach dem erfindungsgemäßen Verfahren ermittelten Daten an den Schaft angebaut, wobei etwaige Anomalien bei Beinprothesen, z.B. O- oder X-Beinigkeit des Patienten, zusätzlich berücksichtigt werden können. Für allfällige Korrekturen der dann sich ergebenden kosmetischen Gestaltung, können im Wege der Optimierung zwischen Kosmetik und Gebrauchsfähigkeit des Stumpfbettes, die entsprechenden Maßnahmen gewählt werden. Mittels einer bekannten Zusatzeinrichtung können danach, vorzugsweise in einer Lage, die aus der Istkurve ermittelbar ist, digitalisierte Daten, welche das Stumpfbett computergerecht beschreiben, gewonnen werden. Die Grenzwerte der Bewegung sind dabei jene Winkel, bis zu deren Erreichen der Schaft vom Patienten schmerzfrei bewegt werden kann. Bei Ausübung des Verfahrens gibt der Patient unbeeinflußt und nicht beobachtbar seine Schmerzempfindungen an das Meßsystem, wodurch die Grenzlage der Prothese in der betreffenden Bewegungsrichtung festgelegt ist.

Die Vorrichtung zur Ausübung des erfindungsgemäßen Verfahrens kennzeichnet sich dadurch, daß die Tragvorrichtung für den Prothesenschaft bevorzugt justierbar an einem Halter angeordnet ist, der seinerseits schwenkbar an einem Tragarm angeordnet ist, der um eine winkelig, insbesondere rechtwinkelig zur Schwenkachse des Halters des Prothesenschaftes angeordnete Achse an einem Schlitten schwenkbar gelagert ist, der an einem ortsfesten, insbesondere von einer Standplatte aufragenden Steher höhenverstell- und feststellbar geführt ist und daß gegebenenfalls die am Schlitten angeordnete Schwenkachse des Tragarmes in einer zum Steher senkrechten Ebene verstellbar im Schlitten geführt ist. Hiedurch kann in einfacher Weise die Vorrichtung hinsichtlich der Lage des Achsenschnittpunktes auf die Lage der Gelenkmitte, bei Beinprothesen auf die Hüftgelenksmitte des jeweiligen Patienten eingestellt werden. Durch Verstellen der am Schlitten angeordneten Schwenkachse in einer zum Steher senkrechten Ebene kann die Vorrichtung an unterschiedliche Hüftweiten angeglichen werden. Die Fehler, die durch Mängel in der Berücksichtigung der Hüftweite entstehen, sind jedoch eher gering. Die vorgenannte Einstellmöglichkeit wird weiters verbessert, wenn die Tragvorrichtung für den Prothesenschaft verschiebbar im Halter geführt und am Halter feststellbar ist. Die Bewegung des Fußes in der Standebene kann simuliert werden, wenn in weiterer Ausgestaltung der Erfindung der Prothesenschaft in der Tragvorrichtung um eine Achse schwenkbar gelagert ist, welche in einer Normalebene auf die Schwenkachse des Halters angeordnet ist. Es ist nicht erforderlich, daß diese Achse genau durch die Gelenksmitte verläuft. Etwaige Abweichungen von der Ideallage beeinflussen das Ergebnis, d.h. die Form und Lage der Nutzfläche nur geringfügig. Im Hinblick darauf kann daher die Schwenkachse unverschiebbar im Halter gelagert werden. Der Prothesenschaft kann hiebei in einfacher Weise in die richtige Lage bezüglich der Mitte des jeweiligen Gelenks, bei Beinprothesen ist dies das Hüftgelenk, gebracht werden, wenn der Prothesenschaft in der Tragvorrichtung auf zwei winkelig, insbes. rechtwinkelig zueinander angeordneten Gleitführungen verstellbaren Tischen gelagert ist, von welchen einer um die Schwenkachse drehbar ist und der andere Tisch den Prothesenschaft trägt.

Um die Grenzlagen, bis zu welchen eine schmerzfreie Bewegung des Schaftes mittels des in den Schaft eingesetzten Stumpfes, insbesondere des Oberschenkelstumpfes möglich ist, festzuhalten, ist in weiterer Ausgestaltung der Erfindung vorgesehen, daß mindestens einer, bevorzugt jedoch allen Drehachsen jeweils ein Winkelmeßgeber zugeordnet ist. Eine weitere Ausführung der erfindungsgemäßen Vorrichtung zeichnet sich dadurch aus, daß mindestens einer, bevorzugt jedoch allen Drehachsen, jeweils ein Drehmomentmeßgeber zugeordnet ist und ein Antriebsmotor und/oder eine Bremse (Bremsgenerator) mit mindestens einer Drehachse, bevorzugt jedoch mit jeder Drehachse jeweils ein Antriebsmotor und/oder eine Bremse (Bremsgenerator) kuppelbar ist. Diese Ausgestaltung erlaubt einen vom Einwirken des Patienten unabhängigen Bewegungsvorgang und die Feststellung des Momentes, das zur Bewegung in der vorgegebenen Richtung notwendig ist bzw. des Bremsmomentes, das aufgebracht werden muß, um eine Bewegung zu hindern. Bekannterweise unterliegt der Prothesenschaft bei seiner bestimmungsgemäßen Verwendung auch Stoßbelastungen. Solche Belastungen treten nach dem Aufsetzen des Schuhes am Boden auf. Um auch für diesen Belastungsfall die Schmerzgrenze feststellen zu können, ist in weiterer Ausgestaltung der Erfindung vorgesehen, über einen Stoßgenerator, der Stöße bevorzugt auf den Tragarm des Prothesenschafthalters überträgt, Stoßspitzen auf den Prothesenschaft in Richtung der in der Normalebene auf die Schwenkachse des Halters angeordneten Schwenkachse des Prothesenschaftes zu übertragen.

Die Erfindung wird nachstehend anhand einer Vorrichtung zur Durchführung des erfindungsgemäßen Verfahrens beispielsweise näher erläutert. Es zeigen,

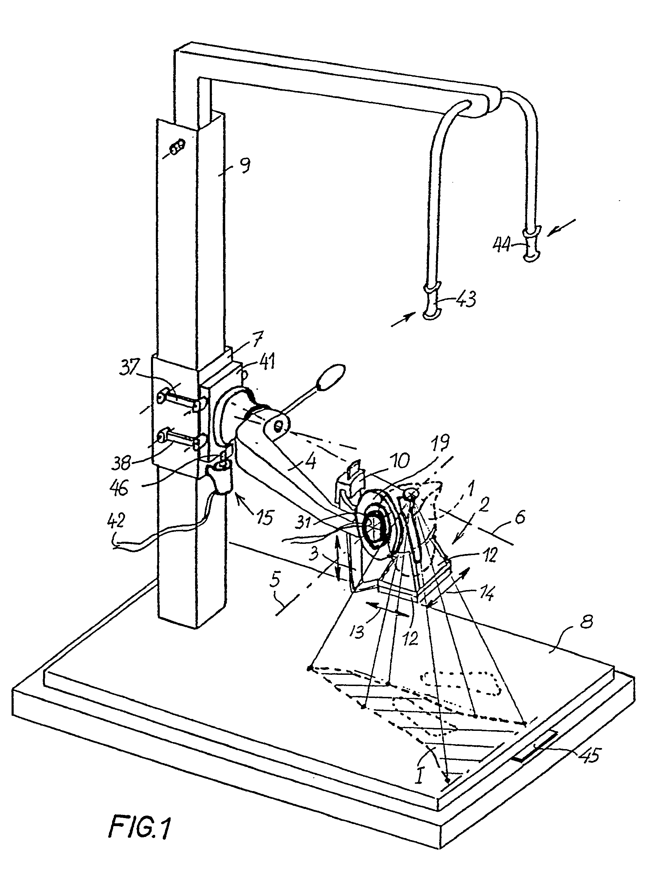

Fig. 1 in schaubildlicher Darstellung, die erfindungsgemäße Vorrichtung mit schematisiert eingetragenem Istbereich der schmerzfreien Bewegbarkeit des Beinstumpfes bei aufgesetztem Prothesenschaft,

Fig. 2 schematisch einen Schnitt entlang der Linie II-II in Fig. 1, wobei zusätzlich zum Istbereich der schmerzfreien Bewegbarkeit des Beinstumpfes auch der Normbereich der Bewegbarkeit eingetragen ist,

Fig. 3 schematisch in Seitenansicht den Prothesenschaft samt Halterung und

Fig. 4 ein Blockschaltbild einer Schaltung für eine erfindungsgemäße Vorrichtung gemäß den Fig. 1 - 3.

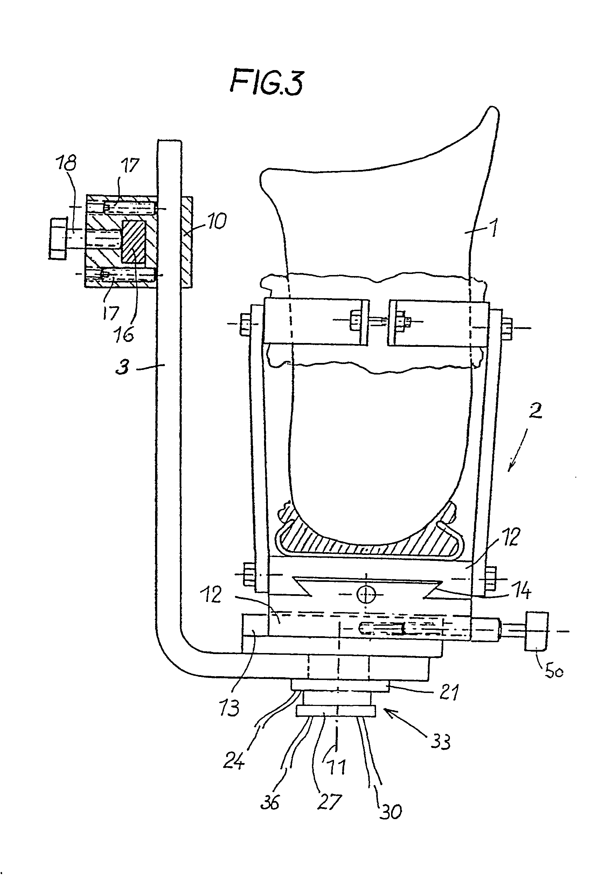

In der Zeichnung ist mit 1 der Prothesenschaft bezeichnet. Der Prothesenschaft 1, der aus Fig. 3 mehr im Detail zu erkennen ist, ist auf einer Trageinrichtung, die als Ganzes mit 2 bezeichnet ist, montiert. Der Halter 2 ist justierbar an einem Tragarm 4 angeordnet, der um eine rechtwinkelig zur Schwenkachse 5 des Halters 3 des Prothesenschaftes 1 angeordnete Achse 6 an einem Schlitten 7 schwenkbar gelagert ist. Der Schlitten 7 kann entlang eines von einer Standplatte 8 aufragenden Stehers 9 in Höhe verstellt und in beliebiger Höhenlage festgestellt werden. Die am Schlitten 7 angeordnete Schwenkachse 6 des Tragarmes 4 kann in einer zum Steher 9 senkrechten Ebene am Schlitten 7 geführt sein und in unterschiedlichen Lagen am Schlitten festgestellt werden. Dies erlaubt bei Oberschenkelprothesen die Berücksichtigung unterschiedlicher Hüftweiten. Die Tragvorrichtung 2 für den Prothesenschaft 1 besitzt einen Bügel 16, an dem in einer Gleitführung 10 der Halter 3 verschiebbar geführt ist. Der Halter 3 ist in der Gleitführung 10 feststellbar. Die Feststellung des Halters 3 in der Gleitführung 10 kann durch zwei Schrauben 17 erfolgen. Die Gleitführung 10 selbst kann entlang des Bügels 16 verschoben und mittels einer Schraube 18 in der jeweils gewünschten Lage am Bügel 16 fixiert werden, der schwenkbar um die Achse 5 am Tragarm 4 gelagert ist.

Der Prothesenschaft 1 ist in der Tragvorrichtung 2, d.h. am Halter 3 der Tragvorrichtung, um eine Achse 11 schwenkbar gelagert. Die Achse liegt in einer Normalebene auf die Schwenkachse 5 des Bügels 16 und damit auch des Halters 3 des Prothesenschaftes 1.

Die Tragvorrichtung 2 besitzt zwei Tische 12, die in zwei winkelig, insbesondere rechtwinkelig zueinander angeordneten Gleitführungen 13, 14 verstellbar sind. Einer der Tische ist dabei um die Schwenkachse 11 drehbar gelagert, wogegen der andere Tisch den Prothesenschaft 1 trägt. Hiedurch ist es möglich, den Prothesenschaft 1 in der Tragvorrichtung zu justieren.

Jeder der Drehachsen 5, 6 und 11 ist jeweils ein Winkelmeßgeber 19, 20, 21 zugeordnet. Die Meßsignale dieser Winkelmeßgeber werden hiebei über Leitungen 22, 23 und 24 abgeführt.

Jeder der drei Achsen 5, 6 und 11 ist weiters jeweils ein Antriebsmotor und/oder eine Bremse 25, 26 und 27 zugeordnet. Die Energiezu- bzw. -abfuhr erfolgt hiebei jeweils über Leitungen 28, 29 und 30. Zur Messung der Drehmomente in den Drehachsen 5, 6 und 11 ist jeder Drehachse jeweils ein Drehmomentenmeßgeber 31 bzw. 32 bzw. 33 zugeordnet. Die Signale deer Drehmomentmeßgeber 31, 32 und 33 werden über Leitungen 34, 35 und 36 abgeführt.

Die Standplatte 8, von welcher der Steher 9 aufragt, kann als Waageplattform ausgebildet sein, wobei mit dieser Platte bevorzugt ein Geber 45 zur Bestimmung des Gewichtes des Patienten dient.

Der Träger der Schwenkachse 6 ist mit dem Schlitten 7 über an jeder Seite des Schlittens 7 angeordnete Paare von Lenkrädern 37, 38 und 39, 40 gekuppelt, die je ein Gelenksparallelogramm bilden. Am Träger 41 der Schwenkachse 6 greift ein Geber 15 für den Druck, der vom Stumpf auf den Schaft 1 übertragen wird, an. Ein Vergleich der Anzeige des Gebers 45 für das Gewicht des Patienten mit der Anzeige des Gebers 15 erlaubt festzustellen, ob der Prothesenschaft voll mit dem Gewicht des Patienten belastet ist, wie dies auch bei der praktischen Benutzung der Prothese der Fall ist. Der Geber 15 kann gleichzeitig auch Teil eines Stoßgenerators zur Übertragung von Stößen auf den Prothesenschaft 1 über den Arm 4 sein. Der Stoßgenerator ist dabei an dem Steher 9 bzw. dem Schlitten 7 fixiert und wird mit elektrischer Energie über Leitungen 42 versorgt, um Stöße auf den Träger 41 der Achse 6 über einen vertikal bewegbaren Zapfen 46 zu übertragen.

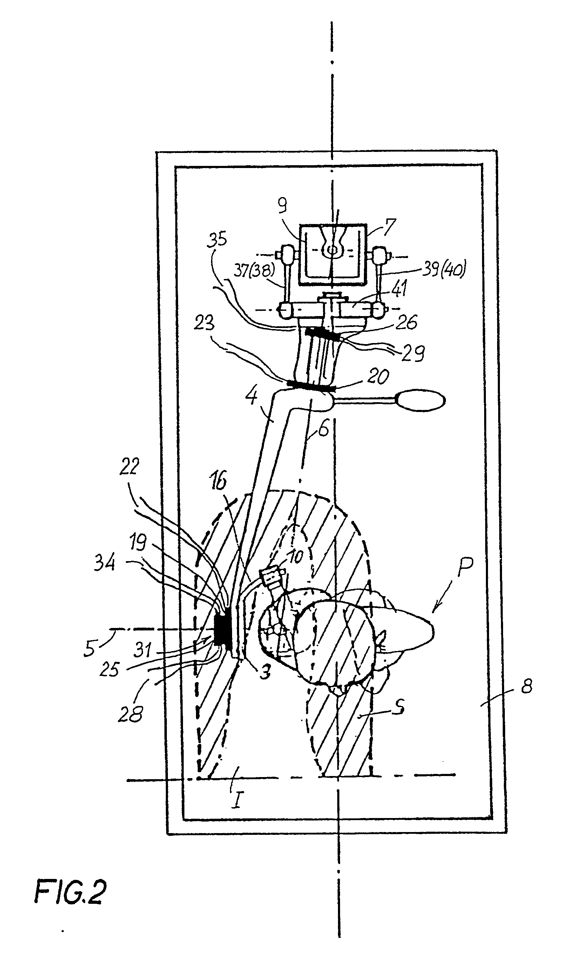

Aus der Draufsichtdarstellung in Fig. 2 ist ersichtlich, daß auf der Standplatte 8 ein Patient P steht und der Prothesenschaft 1 auf einem Oberschenkelstumpf angelegt wurde. Die Nase des Patienten zeigt dabei vom Ständer 9 weg. Am Ständer sind zwei Handgriffe 43, 44 angebracht, die einerseits dazu dienen, dem Patienten P Halt zu geben, anderseits kann durch Gegeneinanderbewegen der Handgriffe 43 und 44 der Patient P ein Signal auslösen, sobald sich bei der Bewegung des Prothesenschaftes 1 Schmerzempfindungen einstellen. Der Patient schwenkt dabei mit seinem Oberschenkelstumpf den Prothesenschaft 1 vor und zurück (bezüglich des Stehers 9) und auch nach links und rechts. Die Schwenkung nach vorne und hinten erfolgt hiebei um die Achse 5 und die seitliche Verschwenkung um die Achse 6. Wie bereits erwähnt, gibt der Patient, sobald sich bei diesen Schwenkbewegungen Schmerzen einstellen, Signale beispielsweise durch Gegeneinanderbewegen der Handgriffe 43 und 44. Jedem Signal kann dabei eine bestimmte Winkelstellung des betreffenden Winkelmeßgebers 20 bzw. 19 und durch den Drehmomentenmeßgeber 31 und 32 auch ein bestimmtes Drehmoment zugeordnet werden. Durch Projektion der relativen Auslenkungen des Prothesenschaftes auf die Bezugsfläche 8 (im Falle einer Beinprothese ist dies die Standfläche) ergibt sich dann eine Istfläche I. Das Zentrum der Projektion ist hiebei die Mitte des natürlichen Gelenks (Hüftgelenks) des Patienten, das dem mit dem Prothesenschaf 1 versehenen Stumpf (Oberschenkelstumpf) zugeordnet ist. Die Istfläche I kann dann mit einer Sollfäche S, sowohl was die Form als auch die Relativlage zur Sollfläche S betrifft, verglichen werden. Die Sollfläche S ist durch genormte Werte für den anzustrebenden Bewegungsbereich des Prothesenschaftes 1 bestimmt. Jeder Stelle der Berandung der Istfläche I ist ein bestimmter Punkt der Berandung der Sollfläche S zugeordnet. Die Ursache, daß die Berandung der Sollfläche S nicht erreicht wird, ist darin gelegen, daß der Bewegungsbereich des Stumpfes durch die Ausführung des Stumpfbettes eingeengt ist. Durch entsprechende Änderung der schmerzverursachenden Stellen im Stumpfbett wird dann eine Verbesserung erzielt und erreicht, daß sich die Berandung der Istfläche I an die Berandung der Sollfläche S nähert. Durch Verstellen des Stumpfes im Halter 3, z.B. durch Kippen, wird auch der Ort der Istkurve I geändert, wodurch etwa eine Äquidistanz der Istkurve zur Sollkurve erzielt werden kann. Damit ist dann auch die Lage des Schaftes zu den damit zu verbindenden mechanischen Elementen der Prothese festgelegt.

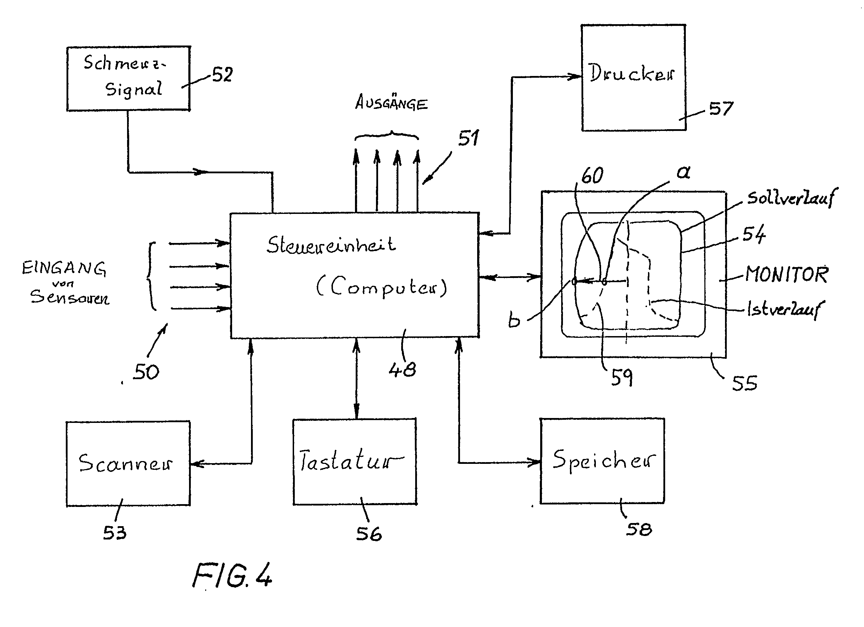

Aus Fig. 4 ist ersichtlich, daß die Ausgänge der Sensoren einem Steuergerät (Computer 48) zugeleitet werden. Diese Eingänge sind mit 50 bezeichnet. Die Ausgänge aus der Steuereinheit 48 sind mit 51 bezeichnet und führen zu den Motoren für die Erzeugung der Drehmomente um die vorgenannten Achsen und zum Stoßgenerator 15. Über einen zusätzlichen Eingang 52 werden die vom Patienten bei Auftreten von Schmerzempfindungen generierten Signale der Steuereinheit 48 zugeleitet.

Der Computer 48 steht mit einem Scanner 53 in Verbindung, der es ermöglicht, eine Sollwertkurve 54 in den Computer einzuspielen, der diese Kurve auf einem Bildschirm 55 wiedergibt. Über eine Tastatur 56 können Daten betreffend die Sollbewegungsweite in jeder Richtung ebenfalls in den Computer 48 eingegeben werden.

Ein Drucker 57 ist vorgesehen, um die durch die erfindungsgemäße Vorrichtung erhaltenen Daten auszudrucken und auch Korrekturen für den Schaft darzustellen, die benötigt werden. Der Computer 48 steht weiters mit einem Speicher 58 in Verbindung, in welchen empirische Daten gespeichert werden können, die sich aus einer bestimmten Koordinatendifferenz zwischen einer Soll- und der zugehörigen Iststelle ergeben, wobei auch die spezielle Korrektur des Prothesenschaftes zugeordnet werden kann. Sobald die Kurve 59 der tatsächlich aufgetretenen Istwerte vorliegt, besitzt ein Momentanvektor 60, der zur Stelle a der Kurve führt, die Polarkoordinaten $(n,\pi)$, während der zugehörige Sollwert b die Polarkoordinaten $(n + e,\pi)$ besitzt. Die Differenz E wird durch den Computer ermittelt und der Computerausdruck zeigt an, daß eine Korrektur e eine Modifizierung des Schaftes an einer bestimmten Stelle und in einem bestimmten Ausmaß erforderlich macht, um die Mobilität in der betreffenden Richtung zu erhöhen. Nachdem die Änderung am Schaft durchgeführt wurde, kann die Messung wie zuvor beschrieben wiederholt werden.

**Patentansprüche**

1. Verfahren zur Bestimmung von Gebrauchseigenschaften eines Prothesenschaftes, insbes. eines Beinprothesenschaftes, bei der Anprobe des Schaftes unter Verwendung einer Tragvorrichtung für den Schaft, dadurch gekennzeichnet, daß die um mindestens eine, bevorzugt jedoch um mindestens zwei einander schneidende, insbes. aufeinander senkrechte Achsen, schwenkbare Tragvorrichtung des Prothesenschaftes, nachdem der Gliedmaßenstumpf, insbes. Beinstumpf, in den Prothesenschaft eingesetzt wurde, mit der Achse bzw. dem Achsenschnittpunkt auf die Mitte (den Mittenbereich) des natürlichen Gelenkes des Gliedmaßen-, insbes. des Beinstumpfes, bzw. auf den Schwerpunkt des den Schaft Probierenden, justiert wird, wonach der Prothesenschaft mittels des Gliedmaßenstumpfes bewegt wird und die Grenzen der Bewegbarkeit des in den Gliemaßen-, insbes. Beinprothesenschaft eingesetzten Gliedmaßen-, insbes. Beinstumpfes koordinatenmäßig als Istwerte festgehalten werden und daß die Istwerte mit genormten Sollwerten zwecks Bewertung der Bewegungseinschränkung hinsichtlich ihrer Zulässigkeit verglichen werden und daß aufgrund der solcherart jeder Stellung des Prothesenschaftes zuordenbaren Abweichung zwischen Soll- und Istwert, Änderungen beispielsweise im Stumpfbett vorgenommen werden, um die Abweichung zwischen Soll- und Istwert der Bewegbarkeit des Gliedmaßen-, insbes. Beinprothesenschaftes in dieser Stellung zu verringern.

2. Vorrichtung zur Durchführung eines Verfahrens nach Anspruch 1, dadurch gekennzeichnet, daß die Tragvorrichtung (2) für den Prothesenschaft (1), bevorzugt justierbar, an einem Halter (3) angeordnet ist, der seinerseits schwenkbar an einem Tragarm (4) angeordnet ist, der um eine winkelig, insbesondere rechtwinkelig zur Schwenkachse (5) des Halters (3) des Prothesenschaftes (1) angeordnete Achse (6) an einem Schlitten (7) schwenkbar gelagert ist, der an einem ortsfesten, insbesondere von einer Standplatte (8) aufragenden Steher (9) höhenverstell- und feststellbar geführt ist und daß gegebenenfalls die am Schlitten (7) angeordnete Schwenkachse (6) des Tragarmes (4) in einer zum Steher (9) senkrechten Ebene verstellbar im Schlitten (7) geführt ist.

3. Vorrichtung nach Anspruch 2, dadurch gekennzeichnet, daß die Tragvorrichtung (2) für den Prothesenschaft (1) verschiebbar im Halter (3) geführt und am Halter (3) feststellbar ist.

4. Vorrichtung nach Anspruch 2 oder 3, dadurch gekennzeichnet, daß der Halter (3) der Tragvorrichtung (2) für den Prothesenschaft (1) in einer Gleitführung (10) verstell- und feststellbar geführt ist, die an einem Bügel (16), insbes. verstell- und feststellbar angeordnet ist, der um die im Tragarm (4) angeordnete Achse (5) schwenkbar ist.

5. Vorrichtung nach einem der Ansprüche 2 bis 4, dadurch gekennzeichnet, daß der Prothesenschaft (1) in der Tragvorrichtung (2) um eine Achse (11) schwenkbar gelagert ist, welche in einer Normalebene auf die Schwenkachse (5) des Halters (3) angeordnet ist.

6. Vorrichtung nach Anspruch 5, dadurch gekennzeichnet, daß der Prothesenschaft (1) gegenüber der in einer Normalebene auf die Schwenkachse (5) des Halters (3) angeordneten Schwenkachse (11) verstellbar ist, wobei in der Tragvorrichtung (2) in zwei winkelig, insbes. rechtwinkelig zueinander angeordneten Gleitführungen (13,14) verstellbare Tische (12) vorgesehen sind, von welchen einer um die Schwenkachse (11) drehbar ist und der andere Tisch den Prothesenschaft (1) trägt.

7. Vorrichtung nach einem der Ansprüche 2 bis 6, dadurch gekennzeichnet, daß mindestens einer, bevorzugt jedoch allen Drehachsen (5,6,11) jeweils ein Winkelmeßgeber (19,20,21) zugeordnet ist.

8. Vorrichtung nach einem der Ansprüche 2 bis 7, dadurch gekennzeichnet, daß mindestens einer, bevorzugt jedoch allen Drehachsen (5,6,11) jeweils ein Drehmomentmeßgeber (31,32,33) zugeordnet ist und ein Antriebsmotor und/oder eine Bremse (Bremsgenerator 25,26,27) mit mindestens einer Drehachse, bevorzugt jedoch mit jeder Drehachse (5,6,11) jeweils ein Antriebsmotor und/oder eine Bremse (Bremsgenerator) kuppelbar ist.

9. Vorrichtung nach Anspruch 2, dadurch gekennzeichnet, daß die Standplatte (8), von welcher der Steher (9) aufragt, als Waageplattform ausgebildet ist, wobei mit dieser Plattform bevorzugt Geber (45) zur Bestimmung des Gewichtes der die Prothese tragenden Person verbunden sind.

10. Vorrichtung nach einem der Ansprüche 2 bis 9, dadurch gekennzeichnet, daß ein Stoßgenerator (15) vorgesehen ist, über welchen Stöße auf den Prothesenschaft (1) bevorzugt über den Tragarm (4) des Prothesenschafthalters (3) übertragbar sind.

FIG.1

FIG.2

## FIG.3

EP 0 449 799 A1

Schmerz-
Signal — 52

Drucker — 57

AUSGÄNGE

51

EINGANG
von
Sensoren

Steuereinheit
(Computer)

50

48

60   a

Sollverlauf
54

MONITOR

Istverlauf

b

59

55

Scanner — 53

Tastatur — 56

Speicher — 58

FIG. 4

**Europäisches Patentamt**

# EUROPÄISCHER RECHERCHENBERICHT

Nummer der Anmeldung

EP  91 89 0041

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-B-1 291 855  (STURM)<br>* Insgesamt *<br>--- | 1-6,9 | A 61 F    2/76<br>A 61 B    5/11 |
| A | GB-A-2 188 846  (ÖBERG)<br>* Zusammenfassung; Figuren *<br>--- | 1,2 | |
| A | US-A-4 667 685  (FINE)<br>* Zusammenfassung; Figur 4 *<br>--- | 1 | |
| A | DE-U-8 603 970  (BÄHR)<br>* Anspruch 1; Figuren *<br>--- | 7 | |
| A | WO-A-8 804 536  (AL-TURAIKI)<br>* Zusammenfassung; Figuren *<br>--- | 8 | |
| A | GB-A-2 121 688  (U.C.L.)<br>* Anspruch 1; Figuren *<br>--- | 9 | |
| A | US-A-4 416 269  (ENOMOTO)<br>* Zusammenfassung; Figuren 1,6 *<br>--- | 10 | |
| A | DE-C-  867 427  (FEINE)<br>--- | | **RECHERCHIERTE SACHGEBIETE (Int. Cl.5)** |
| A | DE-A-2 027 212  (SCHNUR)<br>----- | | A 61 F<br>A 61 B |

Der vorliegende Recherchenbericht wurde für alle Patentansprüche erstellt

| Recherchenort | Abschlußdatum der Recherche | Prüfer |
|---|---|---|
| DEN HAAG | 03-06-1991 | KLEIN C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

  & : Mitglied der gleichen Patentfamilie, übereinstimmendes Dokument

EPO FORM 1503 03.82 (P0401)